# EUROPÄISCHE PATENTANMELDUNG

(11) **EP 0 873 764 A2**
(43) Veröffentlichungstag der Anmeldung: **28.10.1998**
(21) Anmeldenummer: 98106266.4
(22) Anmeldetag: 06.04.1998
(51) Int. Cl.: A61N 5/06

(54) **Gerät für die künstliche Bräunung der Haut**

(30) Priorität: 08.08.1997 IT TV970117; 24.04.1997 IT TV970049
(71) Anmelder: Sun Club S.r.l., 31100 Treviso (IT)
(72) Erfinder: Röhrich, Helmut Gustav, 31100 Treviso (IT)
(74) Vertreter: Glawe, Delfs, Moll & Partner

(57) **Zusammenfassung**

Gegenstand der Erfindung ist ein Gerät für die künstliche Bräunung der Haut, bestehend aus wenigstens einem ersten Strahlungspaneel für die Emission eines im wesentlichen kontinuierlichen Strahlenspektrums, generiert durch Hochdrucklampen und/oder wenigstens einem Strahlungspaneel für die Emission von Strahlung mit einem Leuchtspektrum im wesentlichen in Streifen, generiert durch Niederdrucklampen, das sich dadurch gekennzeichnet, daß an der Frontseite des besagten ersten und/oder zweiten Paneels wenigstens ein Mittel für die Deformierung und/oder die Konzentration und/oder die Verstärkung besagter Strahlen positionierbar ist.

## Beschreibung

Gegenstand der vorliegenden Erfindung ist ein Gerät für die künstliche Bräunung der Haut.

Es ist ein Bräunungsgerät bekannt, das im wesentlichen aus einem ersten Strahlungspaneel für die Emission eines durch Hochdrucklampen erzeugten, im wesentlichen kontinuierlichen Strahlenspektrums und aus einem zweiten Strahlungspaneel für die Emission eines durch Niederdrucklampen erzeugten, im wesentlichen streifenförmigen Leuchtspektrums besteht.

Das erste und das zweite Paneel sind in diesem Gerät derart angeordnet, daß gleichzeitig auf wenigstens einen Teil des Körpers des Nutzers sowohl Strahlen der einen als auch der anderen Art gesendet werden. Dies erlaubt eine gute Bräunung der Haut. Zweckmäßig sind dabei mehrere Anwendungen über einen gewissen Zeitraum hinweg.

Die Aufgabe der vorliegenden Erfindung besteht in der Schaffung eines Geräts, das eine künstliche Bräunung der Haut, die sich von der natürlichen Sonnenbräunung nicht unterscheidet, durch zeitlich verkürzte Anwendungen mit Geräten ermöglicht, die vom Aufbau her einfach sind und begrenzte Kosten verursachen.

Die Aufgabe, die sich vorliegender Antrag setzt, ist also die, die Nachteile der genannten bekannten Technik zu vermeiden, indem ein Gerät geschaffen wird, das das Erlangen einer möglichst gleichförmigen künstlichen Bräunung ermöglicht, die einer natürlichen Sonnenbräunung entspricht, wobei die gewünschten dermatologischen Wirkungen durch eine gegenüber den herkömmlichen Methoden geringere Anzahl von Anwendungen und kürzere Bestrahlungszeiten erreicht werden. Im Rahmen dieser Aufgabe soll auch ein Gerät geschaffen werden, das die vorbenannten Eigenschaften mit einer einfachen Wirkungsweise und einer verläßlichen Anwendung vereint sowie begrenzte Kosten beim Betrieb verursacht. Ferner soll es ermöglicht werden, die Erfindung auch bei Geräten herkömmlicher Art mit wenigen schnellen und einfachen Änderungen zur Wirkung zu bringen.

Die Lösung dieser Aufgabe und weitere Vorteile werden durch die Merkmale des Anspruchs 1 und zweckmäßigerweise der Unteransprüche erreicht.

Weitere Eigenschaften und Vorteile der Erfindung ergeben sich ausführlicher aus der folgenden Beschreibung einer bevorzugten, jedoch nicht ausschließlichen Ausführung, die in den beigefügten Zeichnungen dargestellt ist. Darin zeigen:

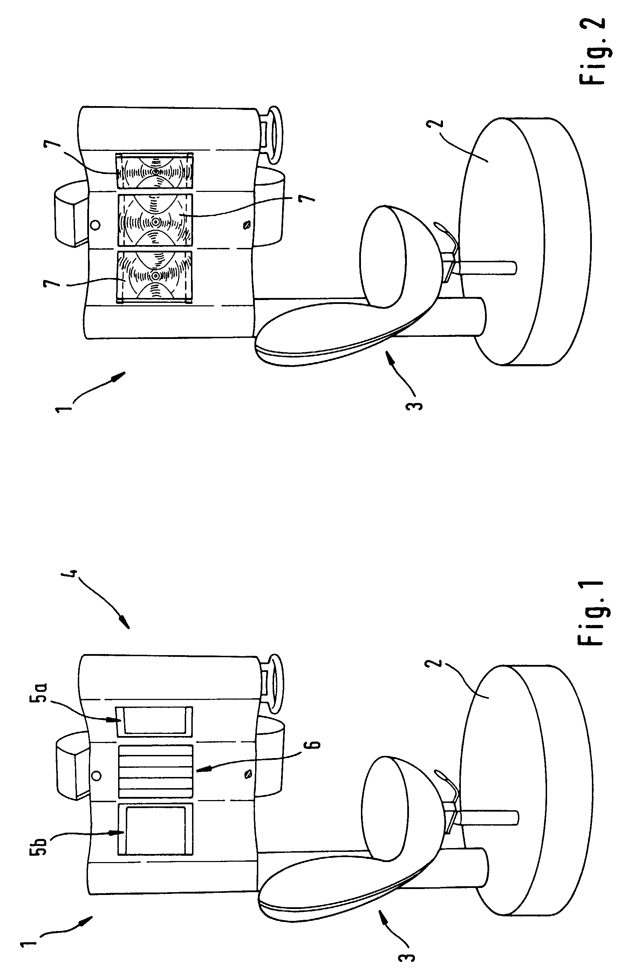

- Fig. 1: ein Gerät herkömmlicher Art,
- Fig. 2: ein erfindungsgemäßes Gerät,
- Fig. 3: in einer Einzelheit die Anwendung des Mittels für die Deformierung und/oder die Konzentration und/oder die Verstärkung der Strahlen in bezug auf das erste oder zweite Paneel,
- Fig. 4: eine weitere Ausführungsform in einer Fig. 1 entsprechenden Darstellung,
- Fig. 5: in einer zur vorhergehenden analogen Sicht eine weitere Ausführungsform, bei der auch eine Mehrzahl von Zylinderlinsen an dem zweiten, zentralen Paneel verwendet wird, und

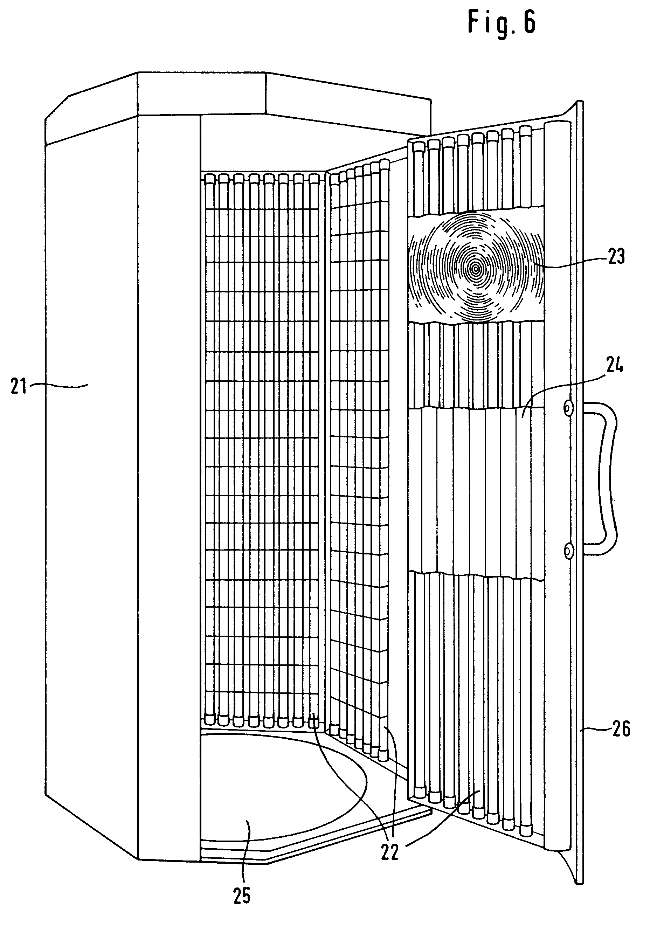

- Fig. 6: in einer zu ¾ seitlichen Ansicht eine Ausführungsform an einem kabinenförmigen Gerät, das für die Aufnahme von einem oder mehreren senkrechten Strahlungspaneelen eingerichtet ist.

Unter Bezugnahme auf die vorbenannten Figuren wurde mit der Bezugsziffer 1 ein Gerät für die künstliche Bräunung der Haut bezeichnet, das beispielsweise aus einem Podest 2 für einen Sitz 3 und einer dreiflügeligen Lampe 4 besteht. Die umfaßt ein Paar von ersten, seitlichen Paneelen 5a, 5b und ein zweites, zentrales Paneel 6. Der Aufbau kann aber auch anders gewählt sein.

Das erste Paar von Paneelen 5a, 5b besteht vorzugsweise aus Hochdrucklampen für die Emission eines im wesentlichen kontinuierlichen Strahlenspektrums. Das zweite, zentrale Paneel 6 besteht vorzugsweise aus Niederdrucklampen für die Emission eines im wesentlichen streifenförmigen Leuchtspektrums.

Die Innovation besteht darin, daß vor den genannten ersten Paneelen und/oder dem zweiten Paneel wenigstens ein Mittel für die Deformierung und/oder die Konzentration und/oder die Verstärkung der von diesen Paneelen erzeugten Strahlen angeordnet ist.

Diese Vorrichtung kann eine Verstärkungs-Eigenschaft hinsichtlich der Richtung oder der Strahlen aufweisen, so daß eine bessere Wirkung bzw. ein verstärkter Strahlungsfluß in Höhe des Gesichtes oder des Körpers des Nutzers erfolgt.

Eine mögliche Ausführungsform dieses Mittels, das allgemein mit der Bezugsziffer 7 bezeichnet ist, kann eine sogenannte Fresnel-Linse sein, die im wesentlichen aus einer Kunststoffolie mit konzentrischen Ringen besteht.

In der dargestellten besonderen Lösung wurden Fresnel-Linsen ausgewählt, da - dank der derzeit verfügbaren Kunststoffe - die gewollten Härte-, Transparenz- und optischen Homogenitäts-Eigenschaften, ähnlich denen von Glas, verfügbar sind. Es werden Kunststoff-Linsen hergestellt, deren Verwendung unter anderem den Vorteil bietet, die Dicke der Linse zu reduzieren, da aufgrund der einzelnen Ringe die Werte auf eine Größenordnung von einem halben Millimeter gebracht werden können. Ein weiterer Vorteil besteht darin, daß die Linsen in optisch bearbeiteten Formen oder Fassungen gegossen werden können, so daß besondere Halterungen oder das Einkitten der Elemente, wie es bei Glaslinsen erforderlich ist, hinfällig werden.

In der dargestellten speziellen Lösung ist das Mittel wegen der durch die ersten Paneele bzw. das zweite Paneel erzeugten Erwärmung in einem gewissen Abstand von diesen angeordnet, so daß ein Luftdurchfluß möglich ist und demzufolge das Mittel eine Temperatur beibehält, das dessen physikalische Eigenschaften nicht verändert.

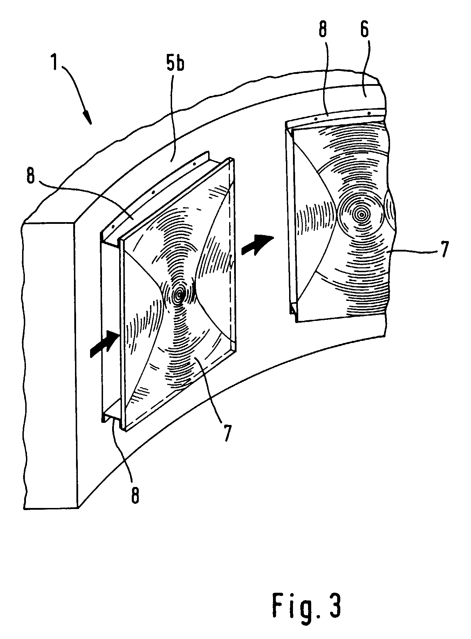

Wie in Fig. 3 dargestellt, kann zu diesem Zweck das gewählte Mittel 3, das aus Plastikfolie besteht, in Höhe der dafür vorgesehenen Aufnahmeführungen oder Halterungen 8 eingesetzt werden und frontseitig zu den ersten beiden Paneelen und/oder dem zweiten Paneel in einem vorgegebenen Abstand zu diesen positioniert werden. Der Abstand zwischen den Paneelen und den Linsen ermöglicht Luftdurchströmung und vermeidet somit eine Überhitzung der Linsen.

Die Erfindung löst die gesetzte Aufgabe und erreicht die vorgegebenen Ziele, indem ein Gerät für die künstliche Bräunung oder dermatologische Behandlung geschaffen wurde, das es ermöglicht, die auf den Körper oder bestimmte Körperpartien gerichtete Strahlung so zu deformieren und/oder zu konzentrieren und/oder zu verstärken, daß sich diese je nach Wunsch auf einen ausgedehnteren Teil des Körpers oder aber nur auf lokal begrenzte Hautpartien auswirken, wobei der Bräunungsgrad oder die dermatologische Heilanwendung mit geringerem Zeitaufwand als bei herkömmlichen Methoden mit gleicher aufgewendeter Leistung erzielt werden können. Dabei kann, je nach den einzelnen Erfordernissen, das geeigneteste Mittel eingesetzt werden.

Die Beschaffenheit dieses Mittels sowie sein Abstand von den ersten Paneelen oder dem zweiten Paneel sind anpaßbar an die jeweiligen Erfordernisse.

Sofern Linsen aus Kunststoff verwendet werden, können Vorrichtungen vorgesehen werden, die eine Luftzufuhr zu den besagten Mitteln erzeugen, so daß diese auf der gewünschten und vorgegebenen Temperatur gehalten werden.

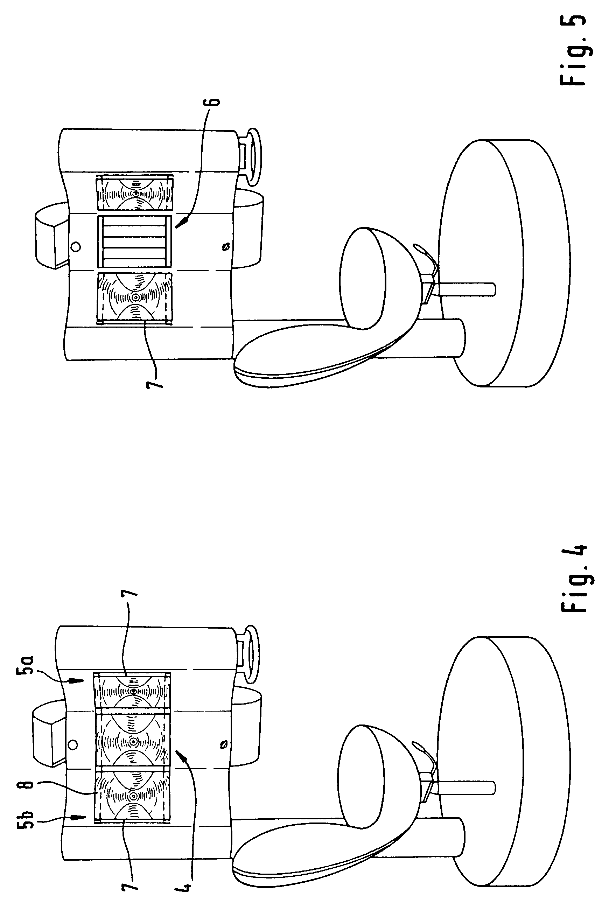

Die Materialien, aus denen die Bausteine dieses Gerätes bestehen, sind entsprechend den jeweiligen Bedürfnissen auswählbar. So können zum Beispiel die Schienen oder Führungen 8, wie sie in Fig. 4 angegeben sind, durchgehend ausgebildet sein, so daß eine kontinuierliche, den ersten bzw. zweiten Paneelen überlagerte Halterung für das optische Mittel gebildet wird.

In Fig. 5 ist eine Variante abgebildet, in der vorzugsweise in Höhe des zweiten, zentralen Paneels 6 ein anderes Mittel für die Deformierung und/oder die Konzentration und/oder die Verstärkung der Strahlung angebracht ist, bestehend aus einer Vielzahl von Zylinderlinsen, die senkrecht aneinandergereiht angeordnet werden.

Ein weiteres Beispiel ist gegeben durch den Einsatz eines Gerätes für die künstliche Bräunung der Haut bestehend aus einer Kabine 21, in welche sich der Nutzer stellt und die eine oder mehrere senkrechte Strahlungspaneele 22 für Strahlung enthält, die in einem im wesentlichen kontinuierlichen Spektrum durch Hochdrucklampen und/oder in einem im wesentlichen linienförmigen Spektrum durch Niederdrucklampen erzeugt wird, wobei vor den besagten senkrechten Paneelen 22 wenigstens ein Mittel angeordnet werden kann für die Deformierung und/oder die Konzentration und/oder die Verstärkung der besagten Strahlen, vorzugsweise in Form einer oder mehrerer Fresnel-Linsen 7 oder Zylinderlinsen 23. Die Länge dieser Linsen kann gleich der Höhe der genannten Kabine oder der genannten senkrechten Paneele oder kürzer sein.

In Höhe des Kabinenbodens ist vorzugsweise mindestens ein die angegebene Strahlung reflektierendes Element vorgesehen, beispielsweise in Form eines runden Podestes 24 mit metallischer Oberfläche vorzugsweise aus Aluminium. Dadurch wird eine verbesserte Bräunung der Beine erreicht. Dieses Merkmal verdient gegebenenfalls Schutz unabhängig von Mitteln für die Deformation, Konzentration oder Verstärkung der Strahlung.

Diese Ausführung erleichtert es, die gesetzten Ziele zu erreichen, und sie ermöglicht dem Nutzer, eine optimale und einheitliche Bräunung in kürzerer Zeit zu erreichen als mit herkömmlichen Methoden.

## Patentansprüche

1. Gerät für die künstliche Bräunung der Haut, beinhaltend wenigstens ein erstes Strahlungspaneel für die Emission von im wesentlichen kontinuierlichen Spektrum-Strahlen, generiert durch Hochdrucklampen und/oder wenigstens einem zweiten Strahlungspaneel für die Emission von Strahlen mit Leuchtspektrum im wesentlichen in Streifen, generiert durch Niederdrucklampen, dadurch gekennzeichnet, daß an der Frontseite des ersten und/oder zweiten Paneels wenigstens ein Mittel für die Deformierung und/oder die Konzentration und/oder die Verstärkung besagter Strahlen positioniert oder eine Einrichtung zur Positionierung eines solchen Mittels vorgesehen ist.

2. Gerät nach Anspruch 1, dadurch gekennzeichnet, daß das Mittel eine Fresnel-Linse umfaßt.

3. Gerät nach Anspruch 1 oder 2, dadurch gekennzeichnet, daß das Mittel eine Verstärkungs-Eigenschaft aufweist in bezug auf die Ausrichtung und/oder Bündelung der durch das erste Paneel und/oder das zweite Paneel erzeugten Strahlen, so daß eine bessere Wirkung oder eine höhere Dichte der Strahlen in Richtung Gesicht oder Körper des Nutzers erzielt wird.

4. Gerät nach einem der Ansprüche 1 bis 3, dadurch gekennzeichnet, daß das Mittel wenigstens eine Fresnel-Linse umfaßt, die im wesentlichen aus einer Plastikfolie mit konzentrischen Ringen besteht.

5. Gerät nach einem der Ansprüche 1 bis 4, dadurch gekennzeichnet, daß das Mittel in einem vorbestimmten Abstand zu dem ersten bzw. zweiten Paneel angeordnet ist, so daß ein Luftfluß dazwischen möglich ist.

6. Gerät nach einem der Ansprüche 1 bis 5, dadurch gekennzeichnet, daß die Einrichtung zur Positionierung des Mittels eine oder mehrere Führungen für das oder die Mittel umfaßt, die das oder die Mittel mit Abstand zu den Paneelen aufnehmen.

7. Gerät nach einem der Ansprüche 1 bis 6, dadurch gekennzeichnet, daß die Führungen durchgehend zur Bildung einer kontinuierlichen Halterung ausgebildet sind, die dem ersten bzw. zweiten Paneel übergelagert ist.

8. Gerät nach einem der Ansprüche 1 bis 7, dadurch gekennzeichnet, daß das Mittel der Höhe wenigstens dieses zweiten, zentralen Paneels in Form einer Mehrzahl von senkrecht, seitlich zueinander angeordneten Zylinderlinsen entspricht.

9. Gerät nach Anspruch 1, dadurch gekennzeichnet, daß es aus wenigstens einer Kabine besteht, die mit einem oder mehreren senkrechten Strahlungspaneelen für die Emission eines im wesentlichen kontinuierlichen, durch Hochdrucklampen erzeugten Strahlungsspektrums und/oder für die Emission eines durch Niederdrucklampen erzeugten streifenförmigen Leuchtspektrums ausgerüstet ist und daß stirnseitig zu den besagten Paneelen wenigstens ein Mittel für die Deformierung und/oder die Konzentration und/oder die Verstärkung besagter Strahlen angeordnet ist oder eine Einrichtung zur Positionierung eines solchen Mittels vorgesehen ist.

10. Gerät nach Anspruch 9, dadurch gekennzeichnet, daß das Mittel für die Deformierung und/oder die Konzentration und/oder die Verstärkung der Strahlen aus einer oder mehreren Fresnel-Linsen und/oder einer oder mehreren Zylinderlinsen besteht.

11. Gerät nach Anspruch 9 oder 10, dadurch gekennzeichnet, daß die Länge dieser Linsen gleich der Höhe der Kabine bzw. der senkrechten Paneele oder geringer ist.

12. Gerät nach einem der Ansprüche 9 bis 11, dadurch gekennzeichnet, daß in Höhe des Kabinenbodens wenigstens ein die Strahlung reflektierendes Element angeordnet ist.

13. Gerät nach Anspruch 12, dadurch gekennzeichnet, daß das reflektierende Mittel aus einem metallischen runden Sockel besteht, vorzugsweise aus Aluminium.
